# EUROPÄISCHE PATENTSCHRIFT

(11) **EP 1 919 360 B1**
(45) Veröffentlichungstag und Bekanntmachung des Hinweises auf die Patenterteilung: **04.05.2011**
(21) Anmeldenummer: 06792624.6
(22) Anmeldetag: 31.07.2006
(51) Int. Cl.: A61B 5/11, A61B 3/113, A61F 2/16

(54) **KÜNSTLICHES AKKOMMODATIONSSYSTEM**
ARTIFICIAL ACCOMMODATION SYSTEM
SYSTEME D'ACCOMMODATION ARTIFICIEL

(30) Priorität: 16.08.2005 DE 102005038542
(43) Veröffentlichungstag der Anmeldung: 14.05.2008
(73) Patentinhaber: Karlsruher Institut für Technologie, 76131 Karlsruhe (DE); Universitäts-Augenklinik Rostock, 18057 Rostock (DE)
(72) Erfinder: BRETTHAUER, Georg, 76139 Karlsruhe (DE); BERGEMANN, Mark, 73033 Göppingen (DE); GENGENBACH, Ulrich, 75196 Remchingen/Singen (DE); KOKER, Torsten, 76297 Stutensee (DE); RÜCKERT, Wolfgang, 42489 Wülfrath (DE); GUTHOFF, Rudolf F., 18119 Rostock (DE)
(74) Vertreter: Fitzner, Uwe
(86) Internationale Anmeldenummer: PCT/EP2006/064869
(87) Internationale Veröffentlichungsnummer: WO 2007/020184

(56) Entgegenhaltungen:
- WO-A-2004/004605
- US-B1- 6 200 342
- US-B2- 6 638 304

## Beschreibung

Gegenstand der Erfindung ist ein künstliches, implantierbares Akkommodationssystem gemäß dem ersten Patentanspruch. Durch die Implantierung eines künstlichen Akkommodationssystems in das menschliche Auge kann die altersbedingt (Presbyopie = Alterssichtigkeit) oder bei einer Kataraktoperation (Grauer Star Operation) verloren gegangene Akkommodationsfähigkeit wiederhergestellt werden.

Das menschliche Auge ist ein optisches System, das mit Hilfe mehrerer lichtbrechender Grenzflächen Objekte scharf auf der Netzhaut (retina) abbildet. Hierbei passieren die Lichtwellen die Hornhaut (cornea), das Kammerwasser in der Vorderkammer (camera anterior bulbi), die Linse (lens crystallina) und den Glaskörper in der Hinterkammer (camera vitrea bulbi), die alle unterschiedliche Brechungsindizes aufweisen. Ändert sich die Gegenstandsweite des betrachteten Objektes, ist es für eine Abbildung mit gleich bleibender Schärfe auf der Netzhaut notwendig, das sich das Abbildungsverhalten des optischen Systems ändert. Beim menschlichen Auge wird dies durch eine Verformung der Linse mit Hilfe des Ziliarmuskels (musculus ciliaris) realisiert, wodurch sich im Wesentlichen die Form und die Lage der Linsenvorder- und -rückseite ändern (Akkommodation). Bei einem intakten Akkommodationssystem eines jugendlichen Menschen kann so die Scheitelbrechkraft des Systems zwischen Ferneinstellung (desakkommodierter Zustand) und Naheinstellung (akkommodierter Zustand) um 14 dpt (Akkommodationsbreite) verändert werden. Dadurch können bei einem normalsichtigen (emmetropen) jugendlichen Menschen Objekte, die sich zwischen dem im Unendlichen liegenden Fernpunkt und dem sich in etwa 7 cm vor der Hornhaut liegenden Nahpunkt befinden, scharf auf der Netzhaut abgebildet werden.

Die Fähigkeit des menschlichen Auges zur Akkommodation nimmt mit zunehmendem Alter ab, wobei ab einem Alter von ca. 50 Jahren keine Akkommodationsfähigkeit (Akkommodationsbreite = 0 dpt) mehr zur Verfügung steht (Presbyopie). Durch den Verlust der Akkommodationsfähigkeit kann das menschliche Auge Objekte in der Nähe (in weniger als ein Meter Entfernung) nicht mehr scharf auf der Netzhaut abbilden. Die Ursachen für die Abnahme und den Verlust der Akkommodationsfähigkeit sind derzeit noch nicht vollständig und eindeutig geklärt; wesentliche Aspekte sind jedoch eine Abnahme der Elastizität der Linse (Linsenverhärtung = lentis sclerosis) und möglicherweise auch eine Verminderung der Kontraktionsfähigkeit des Ziliarmuskels. Bei Ersetzen der getrübten menschlichen Augenlinse durch eine starre Kunstlinse im Rahmen einer Katarakt-Operation geht ebenfalls die Akkommodationsfähigkeit verloren.

Motiviert von den vorgenannten Tatsachen - der Presbyopie und der Katarakt-Operation - sind eine Anzahl von künstlichen implantierbaren Linsensystemen mit variabler Brennweite entwickelt worden.

Bei potentiell akkommodierenden Intraokularlinsen handelt es sich um Linsen oder Linsensysteme, die nach operativer Entfernung der natürlichen Linse anstelle dieser eingesetzt und vorwiegend im Kapselsack befestigt werden. Durch eine noch vorhandene, jedoch geringe Restkontraktion des Ziliarmuskels, soll über eine Haptik eine axiale Verschiebung der Linse erreicht werden.

Beispielhaft wird in DE 94 22 429 U1 eine akkommodierende Intraokularlinse zur Implantation in ein menschliches Auge beschrieben. Sie umfasst einen monolithischen Linsenkörper mit am Umfang angeordneten Stellelementen, welche mit dem Ziliarmuskel des Auges direkt verbunden sind. Zur Akkommodation wird der Linsenkörper durch den Ziliarmuskel über die Stellelemente im Auge vor- und zurückbewegt.

Auch DE 201 11 320 U1 sowie DE 100 62 218 A1 offenbaren implantierbare monolithische Linsen, welche über Stellelemente vom Ziliarmuskel verstellbar sind. Die Stellelemente sind hierbei elastische Hebelelemente, die im unbelasteten Zustand eine Ruheausgangsposition für die Linse definieren.

DE 10139027 A1 beschreibt eine Intraokularlinse mit vorzugsweise vier sich radial nach außen erstreckenden Haptiken, die gelenkig mit der Optik verbunden ist. Durch Kontraktion des Ziliarmuskels soll die Linse mittels des Umlenkmechanismus axial im Strahlengang nach vorne verlagert werden und damit die Brechkraft des Gesamtsystems erhöhen.

Durch derartige Implantate konnte bislang eine axiale Verschiebung der Linse (von maximal 300 µm bis 500 µm) erreicht werden, wodurch eine Akkommodation von 1 dpt bis 2 dpt wiederhergestellt werden kann. Die Gegenstandsweite, bei der Objekte noch scharf auf der Netzhaut abgebildet werden können, beträgt dann beim emmetropen (normalsichtigen) Auge ungefähr 50 cm bis 70 cm. Dies ist jedoch nicht ausreichend, da für Nahsicht (z.B. Lesefähigkeit) eine Akkommodation von > 3 dpt erreicht werden muss. Aus anatomischen Gründen ist jedoch eine dazu notwendige, durch den Ziliarmuskel induzierte Axialverschiebung von mindestens 2 mm nicht möglich. *(*Schneider, H.; Stachs, O.; Guthoff, R.: Evidenzbasierte Betrachtungen zu akkommodativen Kunstlinsen. 102. Jahrestagung der Deutschen Ophthalmologischen Gesellschaft (Berlin, Germany, September 23rd-2Oth 2004) (2004*)); (*Kammann, J.; Dornbach, G.: Empirical results regarding accommodative lenses. In: Current Aspects of Human Accommodation. Hrsg.: Guthoff, R.; Ludwig, K Kaden Verlag Heidelberg (2001) 163-170*).*

WO02/083033 A1 beschreibt eine Linse, bei der die Kontraktion des Ziliarmuskels über eine Verformung des Kapselsackes mehrere Linsensegmente übereinander schieben soll. DE10125829 A 1 beschreibt eine Linse, bei der die Krümmungsradien einer mit einem transparenten Material befüllten Hülle unter Einwirkung des Ziliarmuskels auf den Kapselsack verändert werden sollen. Die beiden letztgenannten Lösungen wurden bislang nicht klinisch erprobt.

Alle genannten Systeme benötigen jedoch einen intakten Ziliarmuskel und einen deformierbaren Kapselsack zur Akkommodation. Steht der Ziliarmuskel nicht mehr oder nur eingeschränkt zur Verfügung, bzw. ist der Kapselsack versteift (Kapselsackfibrose), erfüllen diese Systeme nicht mehr oder nur noch eingeschränkt ihren Zweck.

US 2004/0181279A1 beschreibt eine deformierbare Linse, die nicht auf eine Verformbarkeit des Kapselsacks angewiesen ist. Dabei soll durch eine von der Ziliarmuskelbewegung hervorgerufene axiale Krafteinwirkung zwischen dem steifen, aber durch die Zonulafasern axial beweglichen, Kapselsack und einer außerhalb des Kapselsacks fest fixierten Platte, ein transparenter deformierbarer Körper derart in eine Öffnung dieser Platte gedrückt werden, dass sich dabei innerhalb dieser Öffnung die Krümmungsradien des transparenten deformierbaren Körpers ändern. Eine Erhöhung der Scheitelbrechkraft wäre jedoch bei entspanntem Ziliarmuskel zu erwarten, wenn die Zonulafasern den Kapselsack spannen. Demnach wäre eine physiologische Adaption (Lernprozess, den Ziliarmuskel zur Nahsicht zu entspannen) notwendig. In der Literatur *(*Fine, H.; Packer M.; Hoffmann R.: Technology generates IOL with amplitude of accommodation" (Ophtalmology Times Special Report, March 15th 2005) (2005*))* werden neue Designs, die dieses Problem beheben, in Aussicht gestellt. Auch bei dieser Lösung steht der Nachweis einer klinischen Umsetzbarkeit bislang aus.

Die multifokale Korrektur splittert das einfallende Licht in mindestens zwei Fokuspunkte auf. Die Aufsplittung kann entweder auf refraktiven (Lichtbrechung) oder diffraktiven (Lichtbeugung) Elementen beruhen und rotationssymmetrisch oder segmentförmig ausgeführt sein.

US2002/0149143 A1 beschreibt eine multifokale Intraokularlinse, die das einfallende Licht in mehrere Fokuspunkte aufteilt, so dass eine Sicht in der Ferne und in der Nähe gewährleistet sein soll.

Obwohl Patienten nach einer Lernphase in der Regel tolerieren, dass sich mindestens zwei Bilder auf der Netzhaut überlagern und lernen, ihre bewusste Wahrnehmung auf das scharfe Bild zu richten, ist zwangsläufig eine Verringerung der Kontrastsensitivität in den Fokuspunkten sowie eine erhöhte Blendungsempfindlichkeit bei Punktlichtquellen gegeben (Lavin, M.: Multifocal intraocular lenses - part 1. Optometry Today 5/2001 (2001) 34-37*;* L a vin, M.: Multifocal intraocular lenses - part 2. Optometry Today 8/2001 (2001) 43-44*).*

Eine andere Methode, die sich noch im tierklinischen Versuchsstadium befindet, ist die Befüllung der Linsenkapsel mit einem Polymer, das unter Einwirkung von Licht polymerisiert wird (Lens Refilling). (Nishi, O.; Nishi, K; Mano, C.; Ichihara, M.; Honda, T.: Controlling the capsular shape in lens refilling. Archives of Ophthalmology 115(4) (1997) 501-510*;* Fine, I.H.: The SmartLens- a fabulous neu IOL technology. Eye World 7(10) (2002*)).*

Beim Lens Refilling tritt neben der Schwierigkeit ein geeignetes Polymer zu finden, das u. a. die Anforderungen hinsichtlich Elastizität, Polymerisationsgeschwindigkeit, Brechungsindex, Transmissionsfähigkeit, keine Wasserabsorption, Biokompatibilität erfüllt, das Problem auf, die einzustellende Brechkraft während der Injektion und der Polymerisation zu kontrollieren. Da der Kapselsack nach Entfernung der natürlichen Linse mechanisch versteift (Kapselsackfibrose), ist fraglich, ob Kraft und Kinematik des Ziliarmuskels ausreichen, um eine geeignete Verformung der wiederbefüllten Kapsel zu erreichen.

Ferner wurden technische Gesamtsysteme vorgeschlagen, die die Wiederherstellung der Akkommodationsfähigkeit zum Ziel haben (künstliche Akkommodationssysteme), diese wurden ebenfalls noch nicht klinisch erprobt.

US 6120538 A beschreibt eine Intraokularlinse, auf deren Peripherie mikroelektronische Komponenten montiert sind. Ein Anwendungsbeispiel ist ein künstliches Akkommodationssystem, bestehend aus einer deformierbaren Linse, die von einem Band umschlungen ist, einem Aktor, der die Umschlingungslänge ändern kann und somit eine Linsenverformung verursacht. Die Signalerfassung erfolgt über eine axiale Distanzmessung des Fixationsobjektes oder durch Beobachtung der Schärfe des Netzhautbildes. Über einen geschlossenen Regelkreis wird die Brechkraft des Systems kontinuierlich den Erfordernissen angepasst. Die Energieversorgung des Systems wird mittels eines Photodiodenarrays realisiert.

Die in US 6120538 A vorgeschlagene Lösung substituiert sowohl die Netzhaut als natürlichen Sensor und die natürliche neuronale Informationsverarbeitung des Gehirns. Sie weist den Nachteil auf, dass ein Abstandssensor außerhalb des Strahlengangs positioniert werden muss, um die Distanz zum Fixationsobjekt zu bestimmen. Alternativ ist ein ebenfalls implantierter Bilderfassungssensor zu verwenden, was angesichts des begrenzten Bauraums unrealistisch erscheint. Im Falle einer Beobachtung des Netzhautbildes ist ein relativ rechenintensiver Autofokusalgorithmus zur Maximierung der Bildschärfe einzusetzen. Die Netzhaut ist des Weiteren kein idealer künstlicher Abbildungsschirm, sondern ein mehrlagiges Gewebe aus Photorezeptoren, Nervenzellen und Blutgefäßen.

DE 10155345 C2 beschreibt eine Intraokularlinse, auf deren Aufnahmestruktur Elektrodenstrukturen angeordnet sind, die den ringförmigen Ziliarmuskel elektrisch kontaktieren sollen, um den Sollwert für den notwendigen Akkommodationszustand zu ermitteln. Dieser wird in einem geschlossenen Regelkreis in ein Stellsignal für eine Linse mit variabler Fokuslänge oder eine durch einen Aktor im Strahlengang axial verschiebliche Linse umgesetzt (geschlossener Regelkreis). Die Energieversorgung kann per Fernübertragung über einen wiederaufladbaren Energiespeicher (Batterie, Kondensator) erfolgen.

Ein Nachteil dieser Lösung ist, dass der Ziliarmuskel nur schwer elektrisch zu kontaktieren ist. Bei Implantation des künstlichen Akkommodationssystems im Kapselsack ist damit zu rechnen, dass der Kapselsack als elektrischer Isolator wirkt und keine hinreichend aussagekräftigen Signale erfasst werden können. Der Ziliarmuskel selbst ist durch den Zonulafaserapparat unzugänglich, eine Entfernung von Zonulafasern und Kapselsack ist mit den Qualitätsstandards der Kataraktchirurgie unvereinbar, da die Gefahr einer Destabilisierung des Glaskörpers besteht. Im Spalt zwischen Iris und Ziliarkörper (Sulcus ciliaris) ist davon auszugehen, dass z.B. über einen dort eingelegten Ring keine Muskelaktivität mit Oberflächenelektroden abgreifbar ist.

US6096078 A beschreibt eine Intraokularlinse, die in Ergänzung zu einer herkömmlichen Intraokularlinse in den Sulcus ciliaris implantiert wird und durch die Bewegung des Ziliarmuskels axial verschiebbar sein soll. Sie weist zudem Sensoren auf, die Informationen über den physikalischen Zustand der Linse, insbesondere die mechanische Spannung in der Haptik, ermitteln. Über eine elektrische Schaltung auf der Peripherie der Linse oder auf den Haptiken werden die Informationen an eine externe Auswerteeinheit übertragen. US6096078 A beinhaltet nicht die Idee eines geschlossenen Regelkreises, sondern stellt lediglich eine Möglichkeit zur Erfassung der Ziliarmuskeltätigkeit dar.

US6638304 82 beschreibt eine aus elektrooptischem Material (Flüssigkristalle) bestehende Intraokularlinse mit Elektrodenstrukturen, die das Abbildungsverhalten des optischen Systems verändert. Als mögliche Informationsquellen werden eine Distanzmessung zum Fixationsobjekt (autonomer Autofokus) oder die Nutzung biologischer Signale genannt. Dies umfasst die Registrierung der Steuersignale der Akkommodation: Erfassung der mechanischen Ziliarmuskeltätigkeit über einen Drucksensor, die Erfassung der elektrischen Ziliarmuskelaktivität über Oberflächenelektroden, die Erfassung der Anspannung der Zonulafasern, die Erfassung der Verformung des Kapselsacks. Es wird die Möglichkeit der Registrierung der Steuersignale der Iriskontraktion vorgeschlagen. Ferner wird die Registrierung der Steuersignale der Vergenz genannt: Dazu sollen die Kontraktion eines einzelnen äußeren Augenmuskels (Musculus rectus) mechanisch oder seine neuronale Innervation elektrisch erfasst werden. Über einen Mikrocontroller wird ein geschlossener Regelkreis realisiert. Die Energieversorgung kann über eine Batterie oder eine Fotozelle erfolgen.

Gemäß US6638304 B2 muss die Erfassung der mechanischen Ziliarmuskeltätigkeit über einen Drucksensor außerhalb des Kapselsacks erfolgen. Der Ziliarkörper ist aber, wie bereits dargestellt, durch die Zonulafasern nicht frei zugänglich. Eine Erfassung der elektrischen Ziliarmuskelaktivität über Oberflächenelektroden ist wegen mangelnder Zugänglichkeit und auftretender Elektrodenpassivierung schwer realisierbar. In der US-Patentschrift werden diesbezüglich auch keine Realisierungsvorschläge gemacht. Da nach Entfernung der natürlichen Linse der Kapselsack versteift (Kapselsackfibrose), stellt die Erfassung der Kapselsackverformung keine nutzbare Informationsquelle dar. Die Iriskontraktion allein ist als Informationsquelle nicht geeignet, da sie nicht eindeutig mit der Akkommodation verknüpft ist. Neben dem Pupillennahreflex beeinflusst auch der Pupillenlichtreflex die Pupillenweite.

Im Gegensatz dazu sind Akkommodationsbedarf und Augenmotorik eindeutig miteinander verknüpft. Dazu muss die Orientierung der Fixierlinien beider Augen bestimmt werden. Die Erfassung der elektrischen oder mechanischen Aktivität der horizontalen Augenmuskeln eines einzelnen Auges ist zur Ermittlung der Orientierung der Fixierlinien beider Augen nicht ausreichend, da neben Vergenzen (gegensinnige Rotation der Augen) auch Versionen (gleichsinnige Rotation der Augen) auftreten. Zur Bestimmung der (Horizontal-) Vergenz ist demnach die Differenz der Gierwinkel beider Augen oder alternativ beide Gierwinkel separat (mit anschließender Differenzbildung) zu erfassen. Dies ist in der US-Patentschrift nicht ausgeführt.

WO2004004605 A 1 beschreibt ein künstliches Akkommodationssystem, bestehend aus zwei deformierbaren, sphärischen Linsen für beide Augen, die jeweils von einem Band umschlungen sind, je einem Aktor, der die Umschlingungslänge ändern kann und somit eine Linsenverformung verursacht und je einem Mikrocontroller, der je ein Steuersignal erzeugt, sowie einem auf Messung der Vergenzbewegung basierenden Informationssystem. Drucksensoren, die jeweils zwischen dem nasalen oder temporalen horizontalen äußeren Augenmuskel (Musculus rectus medialis, bzw. Musculus rectus lateralis) und dem Augapfel angebracht sind, erfassen die Flächenpressung in der Grenzfläche. Es wird davon ausgegangen, dass ein Druck nur bei einem Rollen des Augapfels auf die dem Sensor abgewandte Seite entsteht. Ein Steuersignal wird nur dann gesandt, wenn auf beide Sensoren ein Druck ausgeübt wird, d.h. wenn beide Augäpfel eine Innwärts-Rotationsbewegung durchführen. Das Implantat wird von außen mit Energie versorgt.

WO2004004605 A1 benutzt somit als Element, welches das Abbildungsverhalten beeinflusst, eine sphärische elastische Linse, die von einem umschlingenden Band in ihrer Krümmung verändert werden soll. Andere Möglichkeiten der Beeinflussung der Abbildungseigenschaften, die platz- und energiesparender auszuführen sein können, werden nicht erwähnt. Bei der Implantation der Drucksensoren unter die äußeren Augenmuskeln besteht die Gefahr, dass es zu Gewebefibrose verbunden mit einer Gewebeversteifung und Einkapselung des Sensors kommt, wodurch eine Druckmessung unmöglich würde. Es wird ferner davon ausgegangen, dass das Fixationsobjekt sich zentral im Gesichtsfeld befindet, so dass beide Augäpfel eine betragsweise gleichgroße Innwärtsrotation zeigen. Im Allgemeinen tritt jedoch eine Überlagerung von konjugierten (Versionen) und disjungierten (Vergenzen) Augenbewegungen auf. Falls sich das Fixationsobjekt nicht zentral im Gesichtsfeld befindet, unterscheiden sich die Gierwinkel der beiden Augäpfel, so dass nicht zwangsläufig eine Innwärtsrotation beider Augäpfel gegeben ist. Der Einfluss von vertikalen Augapfelbewegungen (Nickbewegungen) auf den Akkommodationsbedarf wird nicht berücksichtigt.

Vorrichtungen zur Wiederherstellung der Akkomodationsfähigkeit sind ferner aus der DE 101 55 345 C2, US 66 38 304 B2, WO 03/017873 A1, US 43,73218 A und US-B1-6200342 bekannt.

Davon ausgehend ist es Aufgabe der Erfindung, ein künstliches, implantierbares Akkommodationssystem vorzuschlagen, das als regelungstechnisches System nicht auf einen Ziliarmuskel als Stellelement, das das optische Abbildungsverhalten beeinflusst, angewiesen ist. Der Ziliarmuskel kann jedoch als Informationsquelle zur Bestimmung des Akkommodationsbedarfs verwendet werden.

Ausgehend von einer Vorrichtung zur Wiederherstellung der Akkommodationsfähigkeit umfassend
a) wenigstens ein optisches System,
b) wenigstens ein zum Ziliarmuskel berührungsloses Informationserfassungssystem entweder mit akustischen oder optischen Mitteln zur Erfassung einer Ziliarkörperbewegung oder mit Mitteln zur Erfassung einer räumlichen Orientierung beider Augäpfel zueinander oder mit Mitteln zur Messung einer Pupillenweite und einer Leuchtdichte mindestens an einem Auge als körpereigenes Steuersignal für die Akkommodation,
c) wenigstens ein Informationsverarbeitungssystem zwecks Erzeugung eines Stellsignals für das optische System aus den erfassten körpereigenen Steuersignalen
d) wenigstens ein Energieversorgungssystem und
e) wenigstens ein Befestigungssystem
wird die Aufgabe der Erfindung dadurch gelöst, dass je ein optisches System, ein Informationserfassungssystem, ein Informationsverarbeitungssystem, ein Energieversorgungssystem und ein Befestigungssystem ein in einen Kapselsack eines Auges integrierbares Implantat bilden.

Die einzelnen Subsysteme sind dabei zu einem, bzw. in mehreren Regelkreisen verschaltet. Das optische System, das Informationserfassungssystem, das Informationsverarbeitungssystem, das Energieversorgungssystem und das Befestigungssystem sind zu einem Implantat zusammengefasst, welches zur Wiederherstellung der Akkommodationsfähigkeit des tierischen oder menschlichen Auges in dieses mittels des Befestigungssystems einsetzbar ist. Hierbei ist das optische System im Strahlengang des Auges angeordnet und bildet mit diesem zusammen den dioptrischen Apparat des Auges. In gleicher Weise sind vorzugsweise das Informationserfassungssystem, das Informationsverarbeitungssystem und das Energieversorgungssystem außerhalb des Strahlengangs angeordnet. Das Informationserfassungssystem kann sich auf mehrere Implantate (z. B. im linken und rechten Augapfel und im Oberkiefer) verteilen. Das Energieversorgungssystem kann vorzugsweise drahtlos mit einem externen System in Verbindung stehen.

Das optische System, bestehend aus einem oder mehreren aktiv-optischen Elementen und / oder einer oder mehreren von Aktoren axial verschieblichen starren Linsen (=passiv-optisches Element), hat die Aufgabe, das Abbildungsverhalten im Strahlengang zu beeinflussen. Es muss im sichtbaren Wellenlängenbereich transparent sein und muss die Lage und / oder die Form mindestens einer seiner lichtbrechenden Grenzflächen zeitlich ändern können, um die Scheitelbrechkraft des dioptrischen Apparates zu verändern. Die aktorische Komponente besteht dabei aus Energiestellern und Energiewandlern *(*Grote / Feldhusen (Hrsg.): Dubbel - Taschenbuch für den Maschinenbau. 21. Auflage. Springer Verlag Berlin Heidelberg New York (2005*)),* welche unter Einwirkung von Stellsignalen einer informationsverarbeitenden Einrichtung Kräfte realisiert, die dann in Bewegung umgesetzt werden können.

Im Falle eines passiv-optischen Elementes werden eine oder mehrere starre Linsen von einem Aktor axial im Strahlengang verschoben. Dieses Wirkprinzip wird standardgemäß in technischen Produkten zur Fokussierung eingesetzt. DE4300840A1 beschreibt z.B. ein Varioobjektiv für Kompaktkameras, das aus zwei Linsengruppen besteht, deren Abstand relativ zueinander variiert werden kann, um eine Brennweitenverstellung durchzuführen.

Verschiedene Mechanismen können zur Erfüllung der oben beschriebenen Aufgabe eines aktiv-optischen Elements zum Einsatz kommen. Dabei ist zwischen einer Änderung der Brechungsindexverteilung und einer Krümmungsänderung einer zwei Medien unterschiedlicher Brechungsindizes trennenden Grenzfläche zu unterscheiden. Diese Veränderungen können durch verschiedene physikalische Wirkungsprinzipien realisiert werden, die im Folgenden dargestellt werden.

Brechungsindexänderung durch elektrooptische Materialien: Die doppelbrechende Eigenschaft elektrooptischer Materialien kann durch elektromagnetische Felder beeinflusst werden. Dadurch kann eine definierte Brechungsindexverteilung eingestellt werden, die eine gezielte Beeinflussung des Abbildungsverhaltens in einer Polarisationsebene des Lichts ermöglicht. Diese kann neben einer gezielten Änderung der Fokuslage auch die Korrektur von Abbildungsfehlern höherer Ordnung (z.B. Astigmatismen, sphärische Aberration, Koma) umfassen. Um beide zueinander senkrechten Polarisationsebenen gleichermaßen zu beeinflussen, ist eine rechtwinklig gekreuzte Hintereinanderanordnung zweier derartiger Systeme notwendig. In US6619799 B1 wird die Verwendung eines derartigen aktiv-optischen Elementes in einem Brillengestell beschrieben. Dabei ist die elektrooptische Schicht von zwei transparenten Elektrodenflächen umfasst, zwischen denen eine elektrische Spannung angelegt werden kann, um das radiale Brechungsindexprofil zu verändern. Ein gewünschtes Brechungsindexprofil kann entweder durch Amplituden- und Frequenzmodulation der Steuerspannung oder durch Aufteilung der Elektroden in mehrere Bereiche, die mit jeweils unterschiedlichen Spannungen versorgt werden, erreicht werden.

Brechungsindexänderung durch Dichteänderung eines kompressiblen Fluids: Der Brechungsindex eines kompressiblen Fluids (z.B. eines Gases oder Gasgemisches) ist von der Dichte abhängig. Diese Abhängigkeit wird durch die Gladstone-Dale-Konstante beschrieben. Werden in einer gasgefüllten Kammer, die ein oder mehrere gekrümmte Begrenzungsflächen aufweist, der Druck und / oder die Temperatur geändert, ändert sich demnach auch das Abbildungsverhalten des optischen Systems. US4732458 A beschreibt z.B. eine derartige Anordnung für ein Mehrlinsenelement, dessen Brechkraft kontinuierlich verändert werden kann. Die Druckerhöhung in der starren gasgefüllten Kammer wird durch einen in einem Zylinder geführten verschieblichen Kolben, der außerhalb der optischen Achse angeordnet ist, realisiert.

Geometrieänderung durch äußere Krafteinwirkung auf einen elastischen Festkörper: Ein elastischer Festkörper, dessen Brechungsindex sich von dem der Umgebung unterscheidet, kann durch Einwirkung äußerer Kräfte so verformt werden, dass sich die Krümmung seiner lichtbrechenden Oberflächen ändert und dadurch das optische Abbildungsverhalten beeinflusst wird. US6493151 B2 beschreibt z.B. eine Anordnung für einen derartig verformbaren homogen oder inhomogen aufgebauten Festkörper, auf den durch einen in seinem Durchmesser veränderbaren Ring radiale Kräfte übertragen werden können. Die Durchmesseränderung des Rings kann thermisch, oder durch magnetische / elektrische Felder erfolgen. DE4345070 A1 beschreibt beispielsweise eine Anordnung für einen verformbaren hüllenförmigen Festkörper, der mit einer lichtdurchlässigen Flüssigkeit gefüllt ist und dessen lichtbrechende Oberflächen über einen ringförmigen Fluidaktor hydraulisch oder pneumatisch verformt werden. DE10244312 A1 nennt als Anwendungsbeispiel für einen Aktor aus Buckypaper (papierartiges Netzwerk von Kohlenstoffnanoröhren) die Änderung der Brechkraft einer künstlichen, in den Augapfel implantierten deformierbaren Linse.

Geometrieänderung durch Benetzungswinkelbeeinflussung (Electrowetting): Zwei ineinander nicht mischbare Fluide annähernd gleicher Dichte, die sich in ihren Brechungsindizes unterscheiden, bilden eine sphärisch gekrümmte oder plane Grenzfläche (Meniskus). Wird das eine, elektrisch leitfähige Fluid, in Kontakt mit einer Elektrode gebracht und gegenüber einer zweiten, von den beiden Fluiden durch eine isolierende Schicht (Dielektrikum) getrennte Elektrode eine Potentialdifferenz angelegt, so lässt sich der Benetzungswinkel und somit die Krümmung des Meniskus durch den sog. Elektrowetting-Effekt ändern. Da der Meniskus zwei Medien unterschiedlichen Brechungsindex trennt, wird das optische Abbildungsverhalten verändert. WO99/18456 A1 beschreibt eine axiale Anordnung von leitfähigem Fluid, transparentem Dielektrikum und transparenter Elektrode im Strahlengang und Maßnahmen zur radialen Zentrierung des Tropfens in der optischen Achse. WO03/069380 A1 beschreibt eine Anordnung, bei der die mit einem Dielektrikum beschichtete Elektrode zylindrisch um die optische Achse angeordnet ist. In der optischen Achse befinden sich axial hintereinander angeordnet das elektrisch-leitfähige Fluid und das isolierende Fluid, sowie der die beiden trennende Meniskus.

Geometrieänderung durch Druckänderung eines Fluides: Wird in einer fluidbefüllten Kammer, die eine oder mehrere deformierbare Begrenzungsflächen aufweist, die Druckdifferenz zur Umgebung geändert, kommt es zu einer Krümmungsänderung der Begrenzungsflächen und demnach auch zur Änderung des Abbildungsverhaltens des optischen Systems. US4466706A beschreibt beispielsweise eine derartige Anordnung, wobei die Druckdifferenzänderung durch einen Verdrängungsmechanismus erreicht wird. Dabei wird durch Drehung einer sich in der zylindrischen Ummantelung befindliche Schraube Fluid verdrängt, was zur Krümmungsänderung der beiden Zylinderendflächen führt. Alternativ kann die zylindrische Ummantelung auch zweiteilig ausgeführt sein, wobei eine Verdrängungswirkung durch eine axiale Relativbewegung beider Teile erzielt werden kann.

Geometrieänderung durch Kraftentwicklung innerhalb eines intelligenten Materials: Intelligente Materialien (Smart Materials) können durch Änderungen ihrer atomaren /molekularen Struktur Kräfte entwickeln und sich dadurch verformen. Durch die Einstellung eines Grenzflächenprofils zwischen intelligentem Material und Umgebung lässt sich demzufolge ebenfalls das optische Abbildungsverhalten beeinflussen. US2004/0100104 beschreibt z.B. einen zu diesem Zwecke verwendeten Formgedächtniskunststoff, der als Phase oder Schicht innerhalb eines verformbaren Linsenkörpers eingebracht ist und die Form des Körpers unter Einwirkung von Energie lokal verändern kann. Als Anwendungsbeispiel wird die postoperative, nicht reversible Korrektur des Abbildungsverhaltens implantierter Intraokularlinsen genannt. JP01230004 beschreibt beispielsweise die Verwendung eines schwellbaren Gels und eines Lösungsmittels, die innerhalb eines deformierbaren Festkörpers schichtartig angeordnet sind. Unter Einwirkung einer Spannung kann die Löslichkeit des Lösungsmittels im schwellbaren Gel so verändert werden, dass dieses daraufhin eine Volumenänderung erfährt. Diese bewirkt eine Krümmungsänderung der lichtbrechenden Oberfläche.

Auch Kombinationen der genannten Wirkprinzipien sind möglich. Das optische System ist folglich in der Lage, die Fokuslage des dioptrischen Apparates anzupassen. Das optische System kann des Weiteren mehrere Elemente umfassen, um das optische Abbildungsverhalten im Strahlengang zu optimieren. Ein enthaltenes aktiv-optisches Element kann gegebenenfalls weitere Abbildungsfehler (monochromatische und chromatische Aberrationen) statisch oder auch dynamisch korrigieren (lokale Beeinflussung der Lichtwellenfront).

Zur Generierung der Stellsignale für die aktorische Komponente des aktiv-optischen Elementes bzw. des passiv-optischen Elementes, ist es notwendig, Informationen zu erfassen, aus denen auf die notwendige Scheitelbrechkrafterhöhung (=Akkommodationsbedarf) geschlossen werden kann.

Informationen über den Akkommodationsbedarf können sowohl aus dem körpereigenen Steuersignal der Akkommodation, als auch aus körpereigenen Steuersignalen der Okulomotorik gewonnen werden. Unter Steuersignalen werden hierbei Informationen verstanden, die den Sollwert oder den unter Einfluss des Sollwertes und möglicher Störsignale umgesetzten Istwert einer Regelstrecke enthalten. Für die Nutzung des Steuersignals der Akkommodation (Nervensignal, Potentialänderung des Ziliarmuskels oder Ziliarmuskelbewegung) kann das Signal eines Auges verwendet werden, da es die vollständige Information über den Akkommodationsbedarf enthält. Werden dagegen die Steuersignale der Okulomotorik, müssen Informationen beider Augen zusammen zur Ermittlung des notwenigen Akkommodationsbedarfs herangezogen werden.

Das biologische Steuersignal der Akkommodation wird vom Edinger-Westphal-Zentrum im Mittelhirn generiert, beide Augen erhalten dabei dasselbe Steuersignal (Prinzip der Konsensualität). Das Signal erreicht jeweils über den Ziliarnerv des parasympathischen Nervensystems in Form einer pulsförmigen Folge von Spannungsschwankungen den Ziliarmuskel des rechten bzw. des linken Auges. Dort innerviert der Nerv den Ziliarmuskel (der zur glatten Muskulatur gerechnet wird) und löst (über die Änderung der Kalziumionenkonzentration in den Muskelzellen) die Kontraktionsbewegung des Ringmuskels aus. Diese Kontraktionsbewegung des Ziliarkörpers, die durch eine radiale Komponente zur optischen Achse hin und eine axiale Komponente von der Iris weg gekennzeichnet ist, kann zur Gewinnung von Informationen über den Akkommodationsbedarf des betreffenden Auges genutzt werden, da die Kontraktionsfähigkeit des Ziliarmuskels im Alter zwar abnimmt, aber auch bei presbyopen Patienten noch nachgewiesen werden kann *(*Stachs, O.: Monitoring the ciliary muscle during accommodation. In: Current Aspects of Human Accommodation II. Hrsg.: Guthoff, R.; Ludwig, K Kaden Verlag Heidelberg (2003) 105-118*).* Der Ziliarkörper wird durch den Kapselsack und den Zonulafaserapparat vom Linseninhalt getrennt. Er ist daher, auch nach Entfernung des Linseninhaltes im Rahmen einer Kataraktoperation, praktisch für eine mechanische oder elektrische Kontaktierung nicht zugänglich.

Daher wird erfingdungsgemäß die berührungslose Erfassung der Ziliarkörperbewegung als Informationsquelle vorgeschlagen. Diese kann z.B. durch eine Abstandsmessung vom Randbereich eines sich im Kapselsack befindlichen Implantates heraus mittels akustischer (z.B. Ultraschall-Laufzeitdifferenz) oder optischer Verfahren erfolgen.

Eine weitere potentielle Informationsquelle stellt die Erfassung der Formveränderung des Sulcus ciliaris dar. Die vorderen, relativ starren Sehnen fixieren den Ziliarmuskel am Sklerafalz und am Trabekelnetzwerk und werden demzufolge bei Kontraktion des Ziliarmuskels auf Zug beansprucht *(*Lütjen-Drecoll, E. Morphology and age-related changes of the accommodation apparatus. In: Current Aspects of Human Accommodation. Hrsg.: Guthoff, R.; Ludwig, K. Kaden Verlag Heidelberg (2001) 25-35*).* Eine Erfassung dieser Sehnenanspannung oder ihrer Auswirkungen auf umliegendes Gewebe kann beispielsweise durch messtechnische Erfassung der Flächenpressung auf einen im Sulcus ciliaris plazierten Ring erfolgen. Dieser kann, muß aber nicht, Bestandteil der Befestigung des Implantates sein.

Eine weitere potentielle Informationsquelle stellt die Erfassung der axialen Kapselsackverschiebung bezüglich mindestens eines außerhalb des Kapselsacks, im Auge gelegenen, Punktes dar. Bei der Kontraktion des Ziliarmuskels verringert sich der Durchmesser dieses Ringmuskels und reduziert auch die Spannung in den Zonulafasern. Ist der Kapselsack im desakkommodierten Zustand in axialer Richtung auf Zug oder Druck vorgespannt, überträgt sich diese Bewegung in Form einer Axialverschiebung des gesamten Kapselsacks im Strahlengang. Diese ist eindeutig mit dem Akkommodationsbedarf verknüpft. Die erforderliche Vorspannung kann beispielsweise durch ein elastisches Material zwischen Kapselsack und einer vor dem Kapselsack im Sulcus ciliaris positionierten Platte erfolgen, die in ihrer Mitte transparent ist. Die Erfassung der Axialrelativbewegung erfolgt dann z.B. durch die Messung der mechanischen Dehnung des eingebrachten elastischen Materials. Eine andere Möglichkeit zur Aufbringung der erforderlichen Vorspannung sind magnetische Kräfte, die zwischen einem Ort im Kapselsack (z.B. einem Teil des Implantates) und einem Ort außerhalb des Kapselsackes (z.B. einer vor dem Kapselsack im Sulcus ciliaris positionierten Platte, die in ihrer Mitte transparent ist) wirken. Auch in diesem Fall können alle physikalisch möglichen Messverfahren (z.B. eine berührungslose optische Abstandsmessung) zur Erfassung der Relativverschiebung zwischen beiden Punkten zum Einsatz kommen.

Die Okulomotorik (vor allem die horizontale Vergenzbewegung) und der Akkommodationsbedarf sind bei den Binokularsehen eindeutig miteinander gekoppelt. Die Fixierlinien beider Augen richten sich durch Rotation der Augäpfel so auf ein beliebig im Raum positioniertes Fixationsobjekt aus, dass das Bild des Fixationsobjektes auf korrespondierende Netzhautstellen fällt. Dadurch wird die Informationsfusion der beiden Einzelbilder zu einem Einfachbild im Gehirn ermöglicht. Die räumliche Orientierung der Augäpfel kann durch die Rotationen der Augäpfel um die drei Raumachsen beschrieben werden. Dabei treten bei jedem Augapfel separat betrachtet Rotationen um die vertikale Achse (Gierbewegungen), um die mitgedrehte horizontale Achse (Nickbewegungen) und die mitgedrehte Fixierlinie (Rollbewegungen) auf. Dementsprechend können in Bezug auf beide Augen konjugierte Augenbewegungen (Versionen = gleichsinnige, gleichgroße Bewegung der Fixierlinien oder der Netzhautmeridiane beider Augen) und disjungierte Augenbewegungen (Vergenzen = gegensinnige, gleichgroße Bewegung der Fixierlinien oder der Netzhautmeridiane beider Augen) unterschieden werden. Im Allgemeinen unterscheiden sich die Abstände des Fixationsobjektes zu den beiden mechanischen Augendrehpunkten und damit auch der Akkommodationsbedarf geringfügig (dies ist insbesondere dann der Fall, wenn sich das Fixationsobjekt nicht symmetrisch zu und nahe an den beiden Augen befindet). Die Erfassung der Steuersignale der Okulomotorik (Nervensignale oder Muskelsignale) ist extrakorporal möglich (z.B. durch die Elektromyogräphie der Augenmuskeln), sie wäre intrakorporal jedoch mit einem hohen Aufwand verbunden. Die Motorik der Augenbewegungen ist auch im hohen Alter hochpräzise und Abweichungen zwischen Soll- und Istwert (=Fixationsdisparität) betragen nur wenige Winkelminuten. Daher ist beim Binokularsehen in sehr guter Nährung ein Schnitt der Fixierlinien gewährleistet und es kann aus den Auswirkungen der Nerven- und Muskelsignale an die Augenmotorik, d.h. aus der Orientierung beider Augäpfel im Raum, auf den Akkommodationsbedarf des rechten, bzw. des linken Auges geschlossen werden. Extrakorporale Verfahren zur Messung der räumlichen Orientierung umfassen u. a. die magnetische Induktionsmethode, bei der mit einer Spule versehene Kontaktlinsen auf die Hornhäute des Patienten aufgebracht oder die Spulen direkt implantiert werden und aus den induzierten Spannungen in einem magnetischen Wechselfeld auf die räumliche Orientierung geschlossen wird oder videobasierte Verfahren, bei denen durch Beobachtung der Pupillen, des Hornhautreflexes oder des Limbus auf die räumliche Orientierung geschlossen wird *(*Joos, M.; Rötting, M.; Velichkovsky, B.M. Die Bewegungen des menschlichen Auge - Fakten, Methoden, innovative Anwendungen. In: Herrmann, T.; Deutsch, S.; Rickheit, G. (Hrsg.), Handbuch für Psycholinguistik De Gruyter Berlin New York (2002) 142-168). Gemäß der Erfindung kann die räumliche Orientierung bezüglich eines kopffesten Bezugssystems (vollständig oder teilweise) durch Abstandsmessung zwischen verschiedenen Bezugspunkten ermittelt werden. Ein kopffestes Bezugssystem bezeichnet ein Koordinatensystem, in dem sich die Lage der beiden mechanischen Augendrehpunkte zeitlich nicht ändert. Aus der gemessenen Orientierung kann wiederum der Akkommodationsbedarf beider Augen bestimmt werden.

Idealerweise wird die räumliche Orientierung beider Augäpfel mittels Abstandsmessung von jeweils einem oder mehreren, exzentrisch zum mechanischen Augendrehpunkt gelegenen augapfelfesten Punkten zu einem oder mehreren kopffesten Bezugspunkten exakt ermittelt. Die augapfelfesten Punkte können sich sowohl im Inneren des Augapfels (z.B. auf dem künstlichen Akkommodationssystem) als auch auf seiner Außenfläche (z.B. auf einer mit dem Augapfel mitbewegten Kontaktlinse oder direkt unter dem Muskelansatz eines äußeren Augenmuskels, Musculus rectus lateralis, Musculus rectus superior, Musculus rectus medialis, Musculus rectus inferior, Musculus obliquus inferior oder Musculus rectus obliquus superior) befinden. Die kopffesten Bezugspunkte sind Punkte, deren räumliche Lage sich im kopffesten Bezugssystem zeitlich nicht ändert (z.B. ein Wangenknochen, der Oberkiefer, nicht jedoch der Unterkiefer).

Auf diese Weise lässt sich der Akkommodationsbedarf des rechten und des linken Auges exakt bestimmen. Zur hinreichend genauen Approximation des Akkommodationsbedarfs kann es aber auch ausreichend sein, eine geringere Anzahl augapfelfester und / oder kopffester Bezugspunkte zu verwenden. Zwei mögliche Anordnungen werden im Folgenden beschrieben:
Wird lediglich der Abstand der beiden auf dem rechten und linken Augapfel befindlichen augapfelfesten Bezugspunkte zueinander bestimmt, kann aus dieser einen Messgröße der Akkommodationsbedarf des rechten und des linken Auges für die Praxis hinreichend genau durch einen einzigen Wert approximiert werden. Eine Unterscheidung zwischen dem Akkommodationsbedarf des rechten und linken Auges ist in diesem Fall (wie auch beim natürlichen Akkommodationssystem) nicht möglich.
Wird jeweils der Abstand eines augapfelfesten Punktes zu einem kopffesten Bezugspunkt bestimmt und der Abstand der beiden augapfelfesten Bezugspunkte untereinander, können aus diesen drei Messgrößen der Akkommodationsbedarf des rechten und des linken Auges getrennt von einander ermittelt werden. Damit kann der Akkommodationsbedarf mit höherer Genauigkeit als mit dem vorigen Verfahren ermittelt werden.

Zur Abstandsmessung zwischen den augapfelfesten Punkten untereinander und / oder einem oder mehreren kopffesten Bezugspunkten können alle geeigneten Messverfahren zum Einsatz kommen. Es kann z.B. die abstandsabhängige Abschwächung eines stationären magnetischen Feldes als Messgröße erfasst werden oder auch die Laufzeitdifferenz oder Phasenverschiebung einer elektromagnetischen Welle.

In der bevorzugten Konfiguration befinden sich in beiden Augenimplantaten Meßsysteme bestehend aus Sender und Empfänger. Im kopffesten Bezugspunkt sitzt ein Objekt, zu dem von einem Implantat aus der Abstand ermittelt wird. Jedes Augenimplantat misst sowohl den Abstand zum Partneraugenimplantat und zu diesem Objekt im kopffesten Bezugspunkt und teilt diese beiden Messwerte dem anderen Augenimplantat mit. Damit stehen jedem Augenimplantat drei Messwerte zur Abstandsmessung zur Verfügung, so dass für den Abstand der beiden augapfelfesten Punkte ein Mittelwert zur Reduktion des Messfehlers gebildet werden kann.

Zur Erhöhung der Zuverlässigkeit der Informationserfassung können redundante Messwerte erfasst werden (z.B. durch Nutzung mehrerer augapfelfester Punkte eines Augapfels).

Alternativ zur Abstandsmessung können bevorzugt die Gierwinkel beider Augen bezüglich des Kopfes über ein geeignetes Meßsystem ermittelt werden. Dafür kommen z.B. Myoelektroden zur Erfassung der Potentialänderungen des Musculus rectus medialis oder lateralis, oder magnetoresistive Sensoren (XMR-Sensoren) auf dem Implantat und ein Referenzmagnet an der Orbitahöhle in Frage.

Die Erfassung der Steuersignale der Pupillenweite allein reicht zur Bestimmung des Akkkommodationsbedarfes nicht aus, da die Messgröße von zwei unbekannten Einflussgrößen (der Leuchtdichte und der Objektdistanz) abhängt. Eine weitere Möglichkeit zur Informationserfassung stellt die gleichzeitige Messung der Pupillenweite bzw. der Iriskontraktions/-dilatationsbewegung und der Leuchtdichte im Strahlengang dar, um die Pupillennahreaktion vom Pupillenlichtrefelex zu trennen. Da die Irisrückseite vom Kapselsack aus zugänglich ist, kann eine Messung der Pupillenweite z.B. auch berührend erfolgen. Alternativ können auch Beleuchtungsdichteunterschiede auf einem Fotodiodenarray hinter der Iris verwendet werden. Gleichzeitig wird der Absolutwert der Beleuchtungsdichte des einfallenden Lichtes vorzugsweise mittels einer Fotodiode erfasst. Aus den Messwerten der Pupillenweite und der absoluten Beleuchtungsdichte kann somit auf den Akkommodationsbedarf geschlossen werden.

Die erfassten Informationen werden im Rahmen der hier beschriebenen Erfindung dem Informationsverarbeitungssystem zur Verfügung gestellt. Gegenstand der Erfindung ist aber auch ein oben beschriebenes Informationserfassungssystem allein, welches Messdaten zur Registrierung und Weiterverarbeitung an einen Empfänger außerhalb des Körpers senden kann.

Die erfassten Signale werden vom Informationsverarbeitungssystem aufbereitet (z.B. Ausreißertests, Glättung, Filterung, Verstärkung). Mit Methoden der klassischen Statistik, der Computational Intelligence und Data Mining werden Merkmale extrahiert und klassifiziert, um die Akkommodationsabsicht zu detektieren. Mit Hilfe von steuerungs- und regelungstechnischen Methoden (z.B. fuzzy-gesteuerter PID-Regler, adaptive Regelungsalgorithmen, selbstlernende Algorithmen) werden die benötigten Stellsignale für das optische System generiert. Es können sowohl hierarchische Regelungsstrukturen als auch zentral-dezentrale Strukturen zum Einsatz kommen.

Zur Versorgung der Subsysteme mit Energie wird ein Energieversorgungssystem eingesetzt, das aus einem Energiewandler, einem Energiespeicher und einer Steuerungseinheit bestehen kann. Der Energiewandler transformiert von außen fernübertragene Energie (z.B. induktiv, kapazitiv, optisch) oder gespeicherte Energie (z.B. Batterie, Miniatur-Brennstoffzelle), die auch in Form von Körperflüssigkeiten (z.B. das nährstoffreiche Kammerwasser, Blut) vorliegen kann, bzw. mechanische Energie (z.B. aus Muskelbewegungen) über einen Energiespeicher in elektrische Energie. Diese wird durch die Steuereinheit des Energieversorgungssystems zu genau definierten Zeitpunkten an die Subsysteme abgegeben. Durch Messung der Beleuchtungsstärke (z.B. durch eine Fotozelle) kann erreicht werden, dass bei Dunkelheit oder bei geschlossenen Augen, d.h. in Situationen, in denen keine Akkommodationsfähigkeit erforderlich ist, das Gesamtsystem in einen Zustand minimalen Energieumsatzes gebracht wird. Die dazu notwendigen Steuersignale werden vom Informationsverarbeitungssystem generiert.

Das Gesamtsystem wird mit Hilfe von geeigneten Befestigungselementen zur axialen Fixierung und radialen Zentrierung im Strahlengang implantiert. Aus der Ophthalmologie (Augenheilkunde) sind für Intraokularlinsen zahlreiche Haptikausführungen bekannt. *(*Draeger, J.; Guthoff, R.F.: Kunstlinsenimplantation. In: Augenheilkunde in Klinik und Praxis Band 4. Hrsg.: Francois, J.; Hollwich, F. Georg Thieme Verlag Stuttgart Nerv York (1991*);* Auffarth, G.U.; Apple, D.J.: Zur Entwicklungsgeschichte der Intraokularlinsen Ophthalmologe 98(11) (2001) 1017-1028*).* Diese finden im Kapselsack Halt.

Das künstliche Akkommodationssystem gemäß der Erfindung ist der technische Teil eines Regelungssystems (geschlossener Regelkreis), welches als künstliches System die Funktion der natürlichen verformbaren Augenlinse und des Ziliarmuskels eines Patienten substituiert. Der biologische Teil besteht im Wesentlichen aus: der Hornhaut, dem Kammerwasser und dem Glaskörper als Bestandteile des dioptrischen Apparates, der Netzhaut als natürlichem Sensorarray und dem Gehirn als natürliche Informationsverarbeitungseinheit, die Steuersignale erzeugt, die Informationen über den Akkommodationsbedarf enthalten.

Das künstliche Akkommodationssystem umfasst ein optisches System, mit einer verstellbaren Brennweite und/oder anderen optischen Eigenschaften. Dieses bildet einen neu eingebrachten Bestandteil des dioptrischen Apparates des Patienten. Es umfasst ein Informationserfassungssystem, das vorzugsweise Abstandsmessungen zwischen augapfelfesten und kopffesten Bezugspunkten ausführt. Auf Basis dieser Abstandmessungen wird der Akkommodationsbedarf durch ein Informationsverarbeitungssystem ermittelt und es werden Stellsignale zum Ansteuern des optischen Systems generiert. Das System wird über ein geeignetes Energieversorgungssystem gespeist und ist über ein Befestigungssystem im Patientenauge fixiert.

Der wesentliche Grundgedanke der Erfindung liegt in einem implantierbaren Gesamtsystem zur teilweisen bzw. vollständigen Wiederherstellung der Akkommodationsfähigkeit, das nicht auf die Kraft des natürlichen Aktors (Ziliarmuskel) angewiesen ist, sondern
(1) entweder die Restaktivität des Ziliarmuskels nutzt, indem es seine Bewegung direkt berührungslos erfasst, bzw. indirekt über die Formänderung des Sulcus ciliaris (hervorgerufen u. a. durch die Anspannung der vorderen Ziliarmuskelsehne), bzw. indirekt über die Axialverlagerung des gesamten Kapselsackes bezüglich eines ortsfesten Punktes außerhalb des Kapselsackes. Im diesem Fall kann ein einzelnes Auge mit einem Implantat ausgerüstet werden.
(2) oder die Koppelung zwischen Akkommodation und Pupillennahreaktion nutzt, wobei aus den Messwerten der Pupillenweite und der absoluten Beleuchtungsdichte auf den Akkommodationsbedarf geschlossen wird. Damit kann der Störgrößeneinfluss des Pupillenlichtreflexes eliminiert werden. Im diesem Fall kann ein einzelnes Auge mit einem Implantat ausgerüstet werden.
(3) oder die Koppelung zwischen Akkommodationsbedarf und Bewegungen des Augenpaares nutzt, ohne in die Motorik der äußeren Augenmuskeln einzugreifen.

In diesem Fall sind folgende Anwendungsmöglichkeiten gegeben:
(a) Bei einer binokularen Implantation wird in beiden Augen ein künstliches Akkommodationssystem implantiert. Als augapfelfeste Bezugspunkte werden bevorzugt Punkte auf dem jeweiligen Implantat gewählt. Die Informationserfassungssysteme der beiden Implantate korrespondieren miteinander, damit eine Information über die Bewegung des Augenpaares vorliegt.
(b) Bei einer monokularen Implantation wird ein Auge mit einem künstlichen Akkommodationssystem versorgt, als augapfelfester Bezugspunkt wird bevorzugt ein Punkt auf dem Implantat gewählt. Zur Bestimmung des Akkommodationsbedarfs ist jedoch ein Bezugspunkt auf dem zweiten Auge notwendig. Um den Augapfel des nicht von der Operation betroffenen Partnerauges nicht öffnen zu müssen, wird ein Hilfsimplantat bevorzugt auf der Außenfläche des Augapfels liegender Bezugspunkt angebracht, das wenigstens ein Informationserfassungssystem umfaßt. Die Informationserfassungssysteme des Implantates und des Hilfsimplantates korrespondieren miteinander, damit eine Information über die Bewegung des Augenpaares vorliegt.

Sowohl bei der monokularen als auch bei der binokularen Implantation kann die Genauigkeit der Approximation des Akkkommodationsbedarfes durch einen kopffesten Bezugspunkt erhöht werden.

Das System ist in der Lage, die Fokuslage wechselnden Gegenstandsweiten anzupassen. Dadurch wird auch ein postoperativer Refraktionsausgleich für unendlich weit entfernte Objekte (Fernpunkt) ermöglicht. Ebenso besteht die Möglichkeit, das System postoperativ wiederholt nachzujustieren z.B. bei Kindern, bei denen sich wachstumsbedingt der notwendige Scheitelbrechkraftbereich ändert.

Bei Einsatz eines geeigneten optischen Systems können zusätzlich auch die sogenannten Aberrationen höherer Ordnung (z.B. Astigmatismen, sphärische Aberration, Koma) korrigiert werden. Grundsätzlich ist die Erfassung dieser Refraktionsdefizite durch eine Wellenfrontanalyse extrakorporal möglich. Die so gewonnenen Informationen können in bestimmten Zeitabständen zur geeigneten Korrektur der Lichtwellenfront verwendet werden.

Durch den Vergleich der Beleuchtungsstärke mit einem Schwellwert, kann der Energieverbrauch des Gesamtsystems in Zuständen, in denen keine Akkommodationsfähigkeit notwendig ist, reduziert werden.

Das beschriebene Akkommodationssystem kann zur Wiederherstellung der Akkommodationsfähigkeit nach Entfernung der natürlichen Augenlinse bei Linsentrübung (Katarakt) oder Alterssichtigkeit (Presbyopie) dienen.

Im Folgenden wird die Erfindung unter Bezugnahme auf die Figuren näher beschrieben:

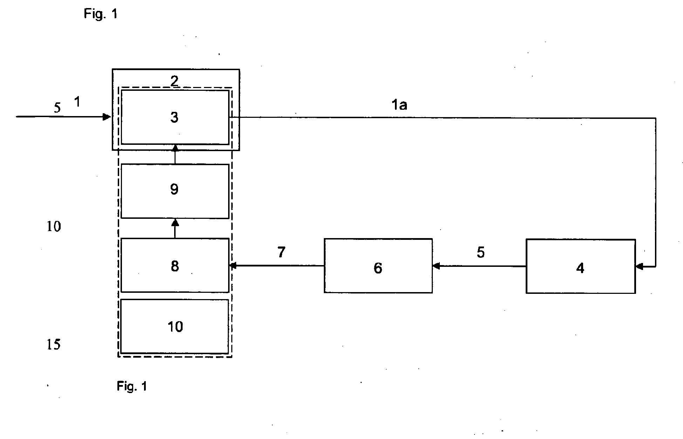

In Figur 1 ist eine schematische Darstellung des Gesamtsystems (künstliches Akkommodationssystem) wiedergegeben. Die Information 1, z.B. Licht von einem Objekt in zeitlich veränderlicher Gegenstandsweite fällt durch den dioptrischen Apparat des menschlichen Auges 2, der das optische System 3 enthält. Das fokussierte Licht 1 a fällt auf den natürlichen Sensor, die Netzhaut 4.

Die durch die Photorezeptoren generierten afferenten Signale 5 werden dem natürlichen Informationsverarbeitungssystem 6, dem Gehirn, zugeleitet. Von dort werden efferente Signale 7, die Information über den Akkommodationsbedarf enthalten, an motorische Strukturen (z.B. Ziliarmuskeln, Bulbusmuskeln) gesendet. Diese Information wird vom Informationserfassungssystem 8 des künstlichen Akkommodations-Systems aufgenommen. Das Informationsverarbeitungssystem 9 leitet daraus Stellsignale für das optische System 3 ab. Damit wird die Scheitelbrechkraft des dioptrischen Apparates 2 durch das künstliche Akkommodationssystem an den Akkommodationsbedarf angepaßt, der aus zeitlich veränderlichen Gegenstandsweiten (Signal 1) resultiert. 10 stellt das Energieversorgungssystem dar. Alle technischen System bestandteile sind durch eine gestrichelte Linie eingerahmt.

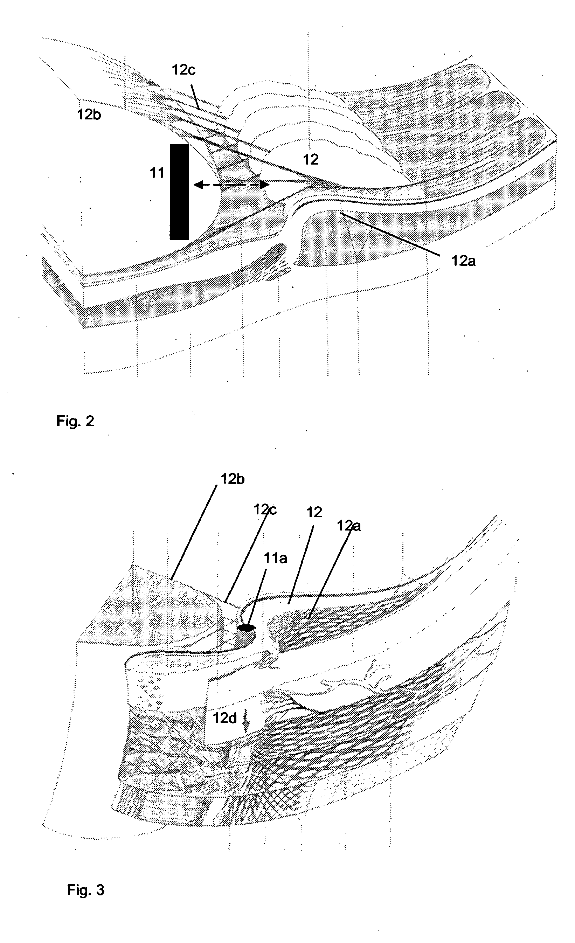

In Figur 2 ist eine Möglichkeit des Informationserfassungssystems 8 im Detail dargestellt. Der Ziliarkörper 12 ist durch den Kapselsack 12b und die Zonulafasern 12c einer mechanischen oder elektrischen Kontaktierung nicht zugänglich. Daher wird die Aktivität des Ziliarmuskels 12a, die sich in einer vorwiegend radialen Verlagerung des Ziliarkörpers 12 äußert, durch ein berührungsloses Abstandsmesssystem 11 erfasst. Dieses bestimmt an einem oder mehreren Punkten des Umfangs den Abstand zum Ziliarkörper 12.

Figur 3 zeigt eine weitere Lösungsmöglichkeit zur Erfassung der Ziliarmuskelaktivität. Die Aktivität des Ziliarmuskels 12a äußert sich neben der oben genannten Verlagerung des Ziliarkörpers 12 auch in einer Anspannung der vorderen Ziliarmuskelsehne 12d, die den Ziliarmuskel im Sclera-Falz fixiert. Diese Anspannung oder ihre Auswirkungen auf umliegendes Gewebe wird durch einen mit geeigneten Sensoren bestückten in den Sulcus ciliaris (zirkuläre Hohlkehle zwischen Iriswurzel und Ziliarzotten) eingelegten Ring 11a erfasst. Zur besseren Illustration sind der Kapselsack 12b und die Zonulafasern 12c ebenfalls in Figur 3 dargestellt.

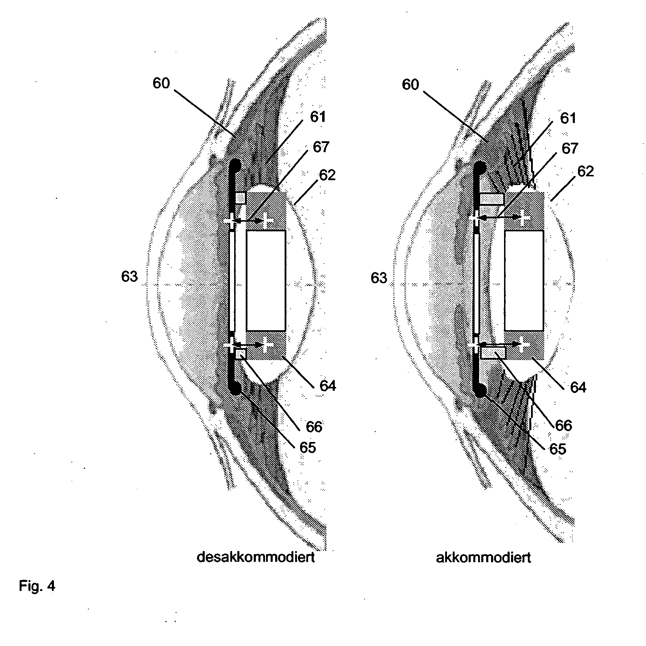

Figur 4 zeigt eine weitere Lösungsmöglichkeit zur Erfassung der Ziliarmuskelaktivität. Der vordere Abschnitt des menschlichen Auges ist im Querschnitt im desakkommodierten (links) und akkommodierten (rechts) Zustand dargestellt. Der im Ziliarkörper gelegene Ziliarmuskel 60 umgibt ringförmig die optische Achse 63. Über die Zonulafasern 61 ist der Kapselsack 62 aufgehängt. 64 stellt einen Teil des Informationserfassungssystems dar, der sich im Kapselsack 62 befindet. 65 stellt einen Teil des Informationserfassungssystems dar, der sich außerhalb des Kapselsacks 62 befindet. Wirkt zwischen beiden Teilen z.B. eine abstoßende Kraft (z.B. magnetisch oder Reaktionskraft eines vorgespannten elastischen Zwischenkörpers 66, auch eine anziehende Kraft ist möglich), so verlagert sich der Kapselsack 62 bei Kontraktion des Ziliarmuskels 60 auf einen geringeren Ringdurchmesser axial nach hinten (rechts). Dadurch vergrößert sich die Distanz 67 zwischen den beiden Bestandteilen des Informationserfassungssystems 64 und 65 im Vergleich zum relaxierten Zustand des Ziliarmuskels (links), der mit einem größeren Ringdurchmesser einhergeht. Durch Erfassung der sich ändernden Distanz kann auf den Kontraktionszustand des Ziliarmuskels und damit auf den Akkommodationsbedarf geschlossen werden.

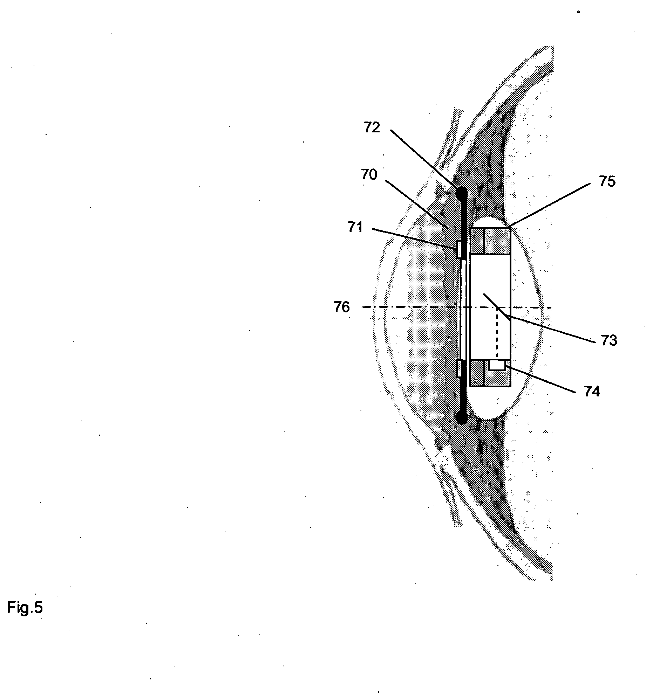

Figur 5 zeigt ein Ausführungsbeispiel eines erfindungsgemäßen Verfahrens der Informationserfassung, bei dem die Koppelung des Akkommodationsbedarfs an die Pupillennahreaktion genutzt wird. Hierzu wird die Kontraktionsbewegung des Irismuskels 70 über einen Sensor 71 berührend erfasst. Dieser ist an einem Element des Befestigungssystems 72 (hier im Sulcus ciliaris plaziert) angebracht, so dass eine Relativbewegung zwischen Sensor und Iris auftritt. Gleichzeitig wird der Absolutwert der Beleuchtungsdichte über eine Photozelle 74 erfasst. Hierzu kann in einem Teil des Strahlengangs ein geeignetes strahlumlenkendes Element 73 angebracht sein, das aber die optische Abbildung auf die Netzhaut längs der optischen Achse 76 nicht behindert. Das strahlumlenkende Element 73 und die Photozelle 74 können, müssen aber nicht, auf einer sich von 72 unterscheidenden Befestigungsstruktur 75 (hier im Kapselsack befestigt) angebracht sein.

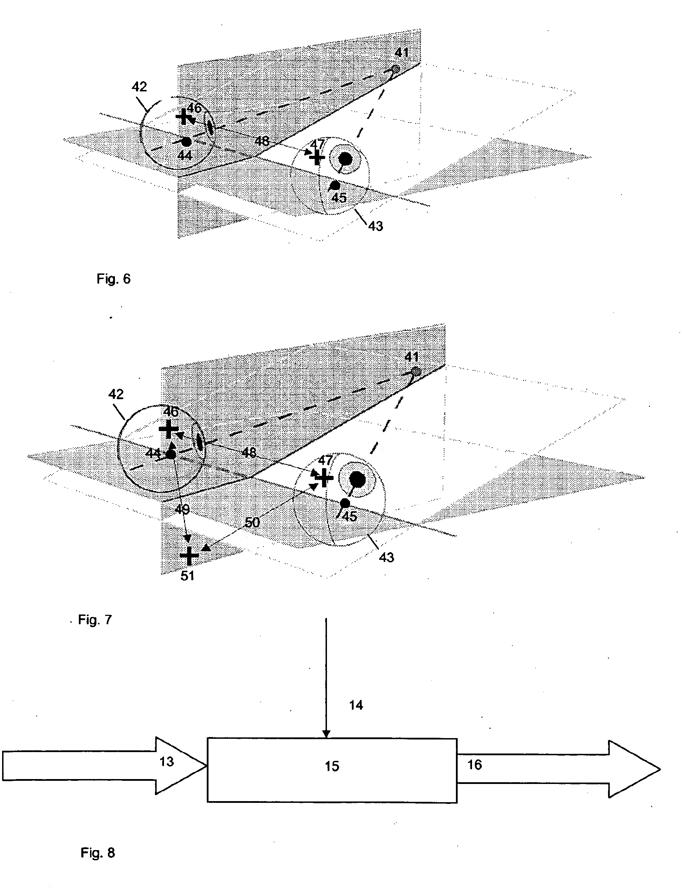

Figur 6 zeigt ein Ausführungsbeispiel eines erfindungsgemäßen Verfahrens der Informationserfassung, bei dem der Abstand 48 zwischen einem augapfelfesten Punkt 46 auf dem linken Augapfel 42 und einem augapfelfesten Punkt 47 auf dem rechten Augapfel 43 ermittelt wird. Die Verbindungslinien zwischen dem Fixationspunkt 41 und den mechanischen Augendrehpunkten 44 bzw. 45 werden als Fixierlinien bezeichnet. Die augapfelfesten Punkte 46 bzw. 47 müssen in Richtung der jeweiligen Fixierlinie einen axialen Abstand zum jeweiligen mechanischen Augendrehpunkt besitzen. Da die augapfelfesten Punkte vorzugsweise nicht im Strahlengang liegen sollen, tritt auch ein radialer Abstand zur Fixierlinie auf.

Figur 7 zeigt ein Ausführungsbeispiel eines erfindungsgemäßen Verfahrens der Informationserfassung, bei dem neben dem in Figur 6 beschriebenen Abstand 48 die Abstände 49 bzw. 50 bestimmt werden. Die Abstände 49 bzw. 50 stellen die Entfernungen zwischen den augapfelfesten Punkten 46 bzw. 47 und einem kopffesten Bezugspunkt 51 dar.

Figur 8 ist eine schematische Darstellung der Funktionen des Energieversorgungssystems. Über die Aufladefunktion 13 wird dem System Energie zugeführt. Hierfür kommen z.B. kapazitive, induktive oder elektromagnetische Wellen in Betracht. Ebenso sind körpereigene Energieressourcen, z.B. mechanische Bewegung von Muskeln, Blut oder Kammerwasser möglich. Das Steuersignal 14 wird der Speicherungs-/Wandlungsvorrichtung 15 zugeführt. Die elektrische Energie 16 wird sodann entladen.

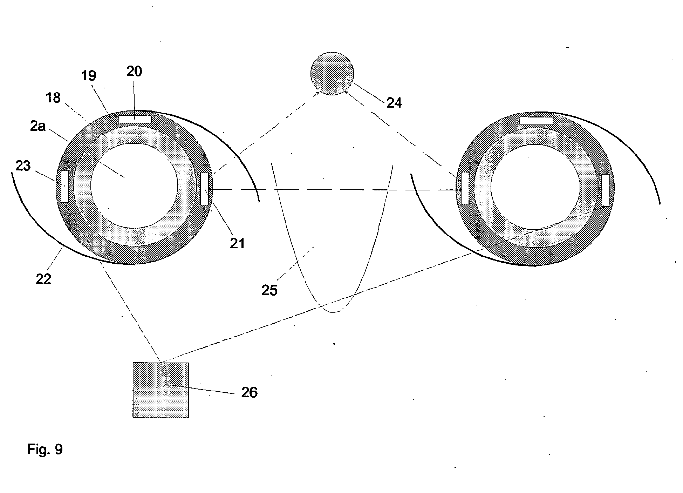

Figur 9 zeigt ein Ausführungsbeispiel eines künstlichen Akkommodationssystems, in Vorderansicht, bei dem in beide Augen ein künstliches Akkommodationssystem implantiert ist (binokulare Implantation).

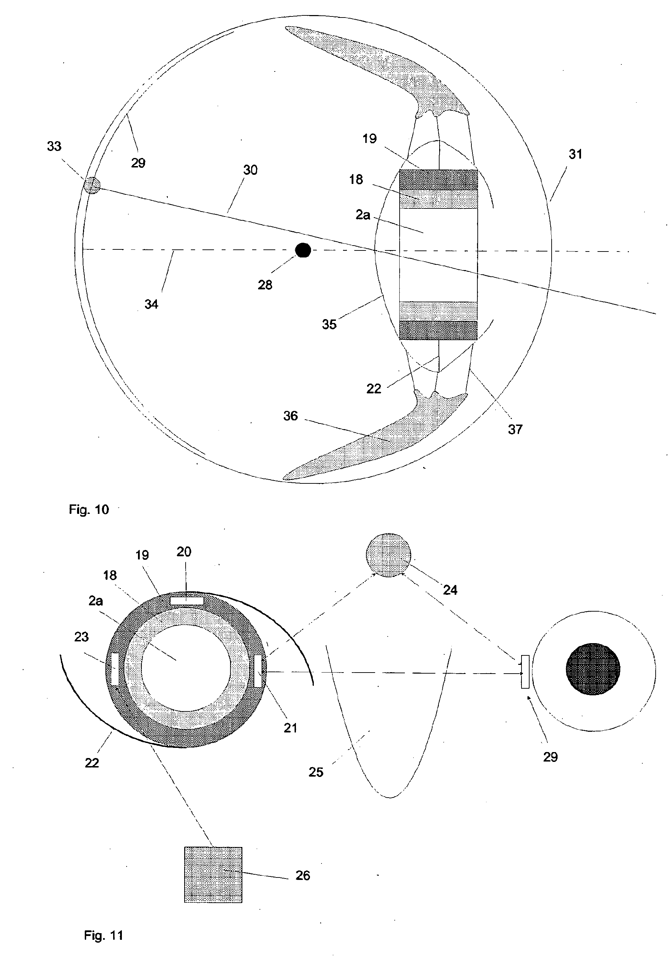

Figur 10 zeigt eine Vergrößerung eines Auges mit einem Ausführungsbeispiel eines künstlichen Akkommodationssystems in Draufsicht.

Das optische System, das Bestandteil des dioptrischen Apparats ist, ist hier ein aktiv-optisches Element. Es ist im Strahlengang hinter der Hornhaut 31 auf der optischen Achse 34 angeordnet. Es besteht aus einer elastischen Linse 2a, um die ringförmig ein Aktor 18 angeordnet ist. In der Bauraumperipherie 19 sind das Informationsverarbeitungssystem 20, ein Teil des Informationserfassungssystems (augapfelfester Punkt 21) und der implantatseitige Teil des Energieversorgungssystems 23 angeordnet. Der kopffeste Bezugspunkt, den beide Informationssysteme nutzen können, ist mit 24 bezeichnet. Er ist beispielsweise über der Nase 25 angeordnet. Das Implantat besitzt Befestigungselemente 22. Ein weiterer Bestandteil des Energieversorgungssystems 26 kann extrakorporal angeordnet sein und Energie an den implantatseitigen Teil 23 übertragen.

In der optischen Achse 34 liegt der mechanische Augendrehpunkt 28. Die Zentralgrube (fovea centralis) 33 befindet sich im Schnittpunkt der Fixierlinie 30 mit der Retina 29. Der Kapselsack 35 wird von dem Ziliarkörper mit Ziliarmuskel 36 und Zonularfasern 37 umgeben. Im Kapselsack 35 ist das Implantat mit Hilfe der Befestigungselemente 22 fixiert.

Figur 11 zeigt ein Ausführungsbeispiel eines künstlichen Akkommodationssystems in Vorderansicht, bei dem in das rechte Auge ein künstliches Akkommodationssystem implantiert ist (monokulare Implantation) und an der Außenseite des Augapfels des Partnerauges ein Hilfsimplantat, das mindestens ein Informationserfassungssystem umfasst, angebracht ist. Mit Ziffer 29 ist der augapfelfeste Bezugspunkt auf dem nicht von der Implantation betroffenen Partnerauge an der Außenfläche des Bulbus dargestellt.

## Patentansprüche

1. Vorrichtung zur Wiederherstellung der Akkommodationsfähigkeit umfassend
a) wenigstens ein optisches System (3),
b) wenigstens ein zum Ziliarmuskel berührungsloses Informationserfassungssystem (8) entweder mit akustischen oder optischen Mitteln zur Erfassung einer Ziliarkörperbewegung oder mit Mitteln zur Erfassung einer räumlichen Orientierung beider Augäpfel zueinander oder mit Mitteln zur Messung einer Pupillenweite und einer Leuchtdichte mindestens an einem Auge als körpereigenes Steuersignal für die Akkommodation,
c) wenigstens ein Informationsverarbeitungssystem zwecks Erzeugung eines Stellsignals für das optische System aus den erfassten körpereigenen Steuersignalen
d) wenigstens ein Energieversorgungssystem (10) und
e) wenigstens ein Befestigungssystem (22).
**dadurch gekennzeichnet, dass** je ein optisches System (3), ein Informationserfassungs- system (8), ein Informationsverarbeitungssystem (9), ein Energieversorgungssystem (10) und ein Befestigungssystem (22) ein in einen Kapselsack eines Auges integrierbares Implantat bilden.

2. Vorrichtung nach einem der vorhergehenden Ansprüche,
**dadurch gekennzeichnet, dass** das optische System (3) ein aktiv-optisches Element mit einer veränderbaren Krümmung einer lichtbrechenden Grenzfläche oder einer veränderbaren Brechungsindexverteilung, ein verschiebbares passiv-optisches Element mit unveränderlichen optischen Eigenschaften oder die Kombination eines oder mehrerer aktiver und/oder passiver optischer Elemente ist.

3. Vorrichtung nach Anspruch 2,
**dadurch gekennzeichnet, dass** das aktiv-optische Element (3) ein elektrooptisches Material, ein kompressibles oder inkompressibles Fluid, einen elastischen Festkörper (2a), ein Benetzungswinkelsystem oder ein aufgrund seiner atomaren und molekularen Struktur innere Kräfte entwickelndes intelligentes Material (18) umfasst.

4. Vorrichtung nach einem der vorhergehenden Ansprüche,
**dadurch gekennzeichnet, dass** das Informationserfassungssystem (8) ein mittels akustischer oder elektromagnetischer oder optischer oder taktiler Verfahren arbeitendes Abstandsmessgerät umfasst.

5. Vorrichtung nach einem der vorhergehenden Ansprüche,
**dadurch gekennzeichnet, dass** das Informationserfassungssystem (8) ein Gerät zur Erfassung einer mechanischen Spannung umfasst.

6. Vorrichtung nach einem der vorhergehenden Ansprüche,
**dadurch gekennzeichnet, dass** ein Akkommodationsbedarf aus dem Steuersignal der Pupillenweite und der absoluten Leuchtdichte im Strahlengang aufgrund der Kopplung von Akkommodation und Pupillennahreflex erhältlich ist.

7. Vorrichtung nach Anspruch 6,
**dadurch gekennzeichnet, dass** das Steuersignal der Pupillenweite mittels Sensor zur Erfassung der Irismuskelbewegung (71) und die Leuchtdichte mit einer Photodiode (74) zur Helligkeitsmessung erfassbar ist.

8. Vorrichtung nach einem der vorhergehenden Ansprüche,
**dadurch gekennzeichnet, dass** ein Akkommodationsbedarf aus dem Steuersignal der Augenmotorik beider Augen aufgrund der Kopplung von Akkommodation und Augenmotorik erhältlich ist.

9. Vorrichtung nach Anspruch 8,
**dadurch gekennzeichnet, dass** zwei miteinander korrespondierende Informationserfassungsysteme (21, 29) das körpereigene Steuersignal der Augenmotorik mittels berührungsloser Bestimmung des Abstands zwischen einem oder mehreren Punkten (46, 47) in oder auf beiden Augäpfeln erfassen.

10. Vorrichtung nach Anspruch 9,
**dadurch gekennzeichnet, dass** ein kopffester Punkt (24, 51) vorgesehen ist, dessen Abstand zu den Punkten (46, 47) in oder auf den Augäpfeln durch die Informationserfassungssysteme (21, 29) berührungslos erfassbar ist.

11. Vorrichtung nach Anspruch 10,
**dadurch gekennzeichnet, dass** zwei miteinander korrespondierende Informationserfassungsysteme das körpereigene Steuersignal der Augenmotorik mittels Bestimmung der Gierwinkel beider Augen bezüglich des Kopfes erfassen.

12. Vorrichtung nach einem der vorhergehenden Ansprüche,
**dadurch gekennzeichnet, dass** das Informationserfassungssystem das körpereigene Steuersignal der Akkommodation aus der Formänderung des Sulcus ciliaris erfasst.

13. Vorrichtung nach Anspruch 12,
**dadurch gekennzeichnet, dass** die Bestimmung der Formänderung des Sulcus ciliaris mittels Messung der Flächenpressung durch einen im Sulcus ciliaris eingelegten Körper (11a) erfolgt.

14. Vorrichtung nach einem der vorhergehenden Ansprüche,
**dadurch gekennzeichnet, dass** das Informationserfassungssystem das körpereigene Steuersignal der Akkommodation aus der Ziliarmuskelkontraktion erfasst.

15. Vorrichtung nach Anspruch 14,
**dadurch gekennzeichnet, dass** die Bestimmung der Ziliar-Muskelkontraktion durch berührungslose Abstandsmessung mit Hilfe eines Sensors (11) erfolgt.

16. Vorrichtung nach einem der vorhergehenden Ansprüche,
**dadurch gekennzeichnet, dass** Mittel zur Beeinflussung einer Verschiebung des Kapselsackes (66) vorgesehen sind und das Informationserfassungssystem das körpereigene Steuersignal der Akkommodation aus der Verschiebung des Kapselsacks erfasst.

17. Vorrichtung nach Anspruch 16,
**dadurch gekennzeichnet, dass** die Mittel zur Beeinflussung einer Verschiebung des Kapselsackes elastische oder magnetische Körper umfassen.

18. Vorrichtung nach Anspruch 16,
**dadurch gekennzeichnet, dass** eine Bestimmung der Verschiebung des Kapselsacks mittels Abstandsmessung (67) erfolgt.

19. Vorrichtung nach Anspruch 18,
**dadurch gekennzeichnet, dass** die Abstandsmessung zwischen einem ortsfesten Punkt im Auge (65) und einem Punkt auf dem Kapselsack (64) erfolgt.

20. Vorrichtung nach einem der vorhergehenden Ansprüche,
**dadurch gekennzeichnet, dass** das Informationsverarbeitungssystem hierarchische Regelstrukturen, zentral-dezentrale Strukturen, mit adaptiven Algorithmen arbeitende Systeme oder mit selbstlernenden Algorithmen arbeitende Systeme oder eine Kombination der genannten Strukturen oder Systeme aufweist.

21. Vorrichtung nach einem der vorhergehenden Ansprüche,
**dadurch gekennzeichnet, dass** die Energieversorgung über ein externes System (26) erfolgt, welches mit dem im Implantat angeordneten Energieversorgungssystem (23) in Verbindung steht.

22. Vorrichtung nach einem der vorhergehenden Ansprüche,
**dadurch gekennzeichnet, dass** das Energieversorgungssystem bei Unterschreitung eines Schwellenwertes der Leuchtdichte im Strahlengang das Akkommodationssystem in einen Zustand minimalen Energieverbrauchs, z. B. Stand-by-Zustand, überführt.

23. Vorrichtung nach einem der vorhergehenden Ansprüche,
**dadurch gekennzeichnet, dass** das Energieversorgungssystem eine interne Energieversorgung aufweist.

24. Vorrichtung nach Anspruch 23,
**dadurch gekennzeichnet, dass** die interne Energieversorgung unter Nutzung körpereigener Ressourcen, Nutzung von Lichtenergie oder technischer Energiespeicher erfolgt.

25. Vorrichtung nach Anspruch 24,
**dadurch gekennzeichnet, dass** das Energieversorgungssystem als technische Energiespeicher Batterien enthält.

26. Vorrichtung nach Anspruch 24,
**dadurch gekennzeichnet, dass** das Energieversorgungssystem zur Nutzung körpereigener Ressourcen Brennstoffzellen enthält.

27. Vorrichtung nach einem der vorhergehenden Ansprüche,
**dadurch gekennzeichnet, dass** das Befestigungssystem (22) eine radiale und axiale Zentrierung im Strahlengang ermöglicht und Verkippungen im Strahlengang vermeidet.

28. Vorrichtung nach Anspruch 27,
**dadurch gekennzeichnet, dass** das Befestigungssystem Befestigungselemente zur axialen und radialen Zentrierung im Strahlengang umfasst.

## Claims

1. Device for restoring the accommodative capacity of the eye, which comprises:
a) at least one optical system (3)
b) at least one data acquisition system (8) that does not touch the ciliary muscle and which has either acoustic or optical means to detect the movement of the ciliary body, or it has means to detect the spatial orientation of the two eyeballs with respect to each other, or it has means for measuring the diameter of the pupil and the luminance at least in one eye as a physiological control signal for accommodation
c) at least one data processing system to produce a regulating signal for the optical system from the physiological control signals received
d) at least one power supply system (10), and
e) at least one system for attachment (22),
**characterized in that** the implant that can be inserted in the capsular sac of an eye is formed integratably by an optical system (3), a data acquisition system (8), a data processing system (9), a power supply system (10) and a system for attachment (22).

2. Device according to any one of the preceding claims, **characterized in that** the optical system (3) is an active optical element with a variable curvature of a light refracting interface or a variable refractive index distribution, a displaceable passive optical element with invariable optical characteristics, or a combination of one or more active and/or passive optical elements.

3. Device according to Claim 2, **characterized in that** the active optical element (3) comprises an electro-optical material, a compressible or incompressible fluid, an elastic solid body (2a), a wetting-angle system or a smart material (18) that generates internal forces due to its atomic and molecular structure.

4. Device according to any one of the preceding claims, **characterized in that** the data acquisition system (8) comprises a distance-measuring apparatus operating by an acoustic, electromagnetic, optical or tactile process.

5. Device according to any one of the preceding claims, **characterized in that** the data acquisition system (8) comprises an apparatus designed for detecting mechanical tension.

6. Device according to any one of the preceding claims, **characterized in that** the need for accommodation can be obtained from the control signal of the diameter of the pupil and the absolute luminance in the light path, using the connection between accommodation and the pupillary near reflex.

7. Device according to Claim 6, **characterized in that** the control signal of the diameter of the pupil can be obtained by a sensor designed to detect the movement of the iris muscle (71), and the luminance can be determined by a photodiode (74) designed to measure the brightness.

8. Device according to any one of the preceding claims, **characterized in that** the need for accommodation can be obtained from the control signal of the motor activity of both eyes, thanks to the connection between accommodation and oculomotor activity.

9. Device according to Claim 8, **characterized in that** two mutually communicating data acquisition systems (21, 29) detect the physiological control signal of the oculomotor activity by the contact-free determination of the distance between one or more points (46, 47) in or on the two eyes.

10. Device according to Claim 9, **characterized in that** a fixed-head point (24, 51) is chosen whose distance from the points (46, 47) in or on the eyeballs can be determined by the data acquisition systems (21, 29) without contact.

11. Device according to Claim 10, **characterized in that** two mutually communicating data acquisition systems detect the physiological control signal of the oculomotor activity by determining the angle of yaw of both eyes with respect to the head.

12. Device according to any one of the preceding claims, **characterized in that** the data acquisition system takes the change in the shape of the ciliary sulcus as the physiological control signal of accommodation.

13. Device according to Claim 12, **characterized in that** the change in the shape of the ciliary sulcus is determined by measuring the surface compression with the aid of a body (11a) inserted into the ciliary sulcus.

14. Device according to any one of the preceding claims, **characterized in that** the data acquisition system detects the physiological control signal of accommodation from the contraction of the ciliary muscle.

15. Device according to Claim 14, **characterized in that** the contraction of the ciliary muscle is determined by contact-free distance measurement carried out with the aid of a sensor (11).

16. Device according to any one of the preceding claims, **characterized in that** a means for influencing the displacement of the capsular sac (66) is provided, and the data acquisition system takes the displacement of the capsular sac as the physiological control signal of accommodation.

17. Device according to Claim 16, **characterized in that** the means for influencing the displacement of the capsular sac comprises elastic or magnetic bodies.

18. Device according to Claim 16, **characterized in that** the displacement of the capsular sac is determined by distance measurement (67).

19. Device according to Claim 18, **characterized in that** it is the distance between a stationary point in the eye (65) and a point on the capsular sac (64) that is measured.

20. Device according to any one of the preceding claims, **characterized in that** the data processing system has hierarchical regulating structures, central/decentral structures, systems operating with adaptive algorithms or systems operating with self-learning algorithms, or it has a combination of these structures or systems.

21. Device according to any one of the preceding claims, **characterized in that** the power is supplied by an external system (26) that is connected to the power supply system (23) arranged in the implant.

22. Device according to any one of the preceding claims, **characterized in that** power supply system puts the accommodation system in a state of minimum energy consumption, e.g. stand-by state, when the threshold value of luminance in the light path is not reached.

23. Device according to any one of the preceding claims, **characterized in that** the power supply system has an internal power supply.

24. Device according to Claim 23, **characterized in that** the internal power supply operates by using both physiological resources and the energy of light or technical energy storage devices.

25. Device according to Claim 24, **characterized in that** the power supply system comprises batteries as technical energy storage devices.

26. Device according to Claim 24, **characterized in that** the power supply system comprises fuel cells for utilizing physiological resources.

27. Device according to any one of the preceding claims, **characterized in that** the system for attachment (22) makes a radial and axial centring in the light path possible and prevents tilting in the light path.

28. Device according to Claim 27, **characterized in that** the system for attachment comprises fixing elements for axial and radial centring in the light path.

## Revendications

1. Dispositif pour rétablir l'aptitude à l'accommodation de l'eil, comprenant :
a) au moins un système optique (3),
b) au moins un système d'acquisition de données (8) ne perturbant pas le muscle ciliaire et comprenant soit des moyens acoustiques ou optiques destinés à détecter un mouvement du corps ciliaire, soit des moyens destinés à détecter une orientation spatiale des deux globes oculaires l'un par rapport à l'autre, soit des moyens destinés à mesurer un diamètre de pupille et une luminance dans au moins un oeil sous la forme d'un signal de commande endogène destiné à l'accommodation,
c) au moins un système de traitement de données destiné à produire un signal de réglage pour le système optique à partir du signaux de commande endogène reçu,
d) au moins un système d'alimentation en énergie (10), et
e) au moins un système de fixation (22),
**caractérisé en ce qu'**un système optique (3), un système d'acquisition de données (8), un système de traitement de données (9), un système d'alimentation en énergie (10) et un système de fixation (22) forment un implant pouvant être intégré au sac capsulaire d'un oeil.

2. Dispositif selon l'une des revendications précédentes,
**caractérisé en ce que** le système optique (3) est un élément optique actif ayant une courbure modifiable d'une surface de séparation réfractante où une répartition modifiable de l'indice de réfraction, un élément optique passif coulissant ayant des propriétés optiques non modifiables, ou la combinaison d'un ou de plusieurs éléments optiques actifs et/ou passifs.

3. Dispositif selon la revendication 2,
**caractérisé en ce que** l'élément optique actif (3) comprend un matériau électro-optique, un fluide compressible ou incompressible, un solide élastique (2a), un système d'angle de mouillage ou un matériau (18) intelligent développant des forces internes en fonction de sa structure atomique ou moléculaire.

4. Dispositif selon l'une des revendications précédentes,
**caractérisé en ce que** le système d'acquisition de données (8) comprend un dispositif de mesure de distance fonctionnant selon un procédé acoustique ou électromagnétique ou optique ou tactile.

5. Dispositif selon l'une des revendications précédentes,
**caractérisé en ce que** le système d'acquisition de données (8) comprend un dispositif destiné à détecter une tension mécanique.

6. Dispositif selon l'une des revendications précédentes,
**caractérisé en ce qu'**un besoin d'accommodation peut être obtenu à partir du signal de commande du diamètre de pupille et de la luminance absolue sur le chemin optique en fonction du couplage entre l'accommodation et le réflexe pupillaire en vision de près.

7. Dispositif selon la revendication 6,
**caractérisé en ce que** le signal de commande du diamètre pupillaire peut être obtenu au moyen d'un capteur destiné à détecter le mouvement du muscle de l'iris (71) et la luminance au moyen d'une photodiode (74) pour la mesure de luminosité.

8. Dispositif selon l'une des revendications précédentes,
**caractérisé en ce que** le besoin d'accommodation peut être obtenu à partir du signal de commande de motricité oculaire des deux yeux en fonction du couplage entre l'accommodation et la motricité oculaire.

9. Dispositif selon la revendication 8,
**caractérisé en ce que** deux systèmes d'acquisition de données (21, 29) correspondant l'un à l'autre détectent le signal de commande endogène de motricité oculaire par détermination sans contact de la distance entre un ou plusieurs points (46, 47) situés dans ou sur les deux globes oculaires.

10. Dispositif selon la revendication 9,
**caractérisé en ce qu'**il est prévu un point (24, 51) fixe par rapport à la tête dont la distance aux points (46, 47) se situant dans ou sur les globes oculaires peut être détectée sans contact au moyen des systèmes d'acquisition de données (21, 29).

11. Dispositif selon la revendication 10,
**caractérisé en ce que** deux systèmes d'acquisition de données correspondant l'un à l'autre détectent le signal de commande endogène de motricité oculaire par détermination de l'angle de lacet des deux yeux par rapport à la tête.

12. Dispositif selon l'une des revendications précédentes,
**caractérisé en ce que** le système d'acquisition de données détecte le signal de commande endogène d'accommodation à partir de la modification de forme du sillon ciliaire.

13. Dispositif selon la revendication 12,
**caractérisé en ce que** la détermination de la modification de forme du sillon ciliaire s'effectue par mesure de la pression de surface au moyen d'un corps (11a) inséré dans le sillon ciliaire.

14. Dispositif selon l'une des revendications précédentes, **caractérisé en ce que** le système d'acquisition de données détecte le signal de commande endogène d'accommodation à partir de la contraction du muscle ciliaire.

15. Dispositif selon la revendication 14,
**caractérisé en ce que** la détermination de la contraction du muscle ciliaire s'effectue par mesure de distance sans contact à l'aide d'un capteur (11).

16. Dispositif selon l'une des revendications précédentes,
**caractérisé en ce que** des moyens sont prévus pour provoquer un déplacement du sac capsulaire (66) et **en ce que** le système d'acquisition de données détecte le signal de commande endogène d'accommodation à partir du déplacement du sac capsulaire.

17. Dispositif selon la revendication 16,
**caractérisé en ce que** les moyens destinés à provoquer un déplacement du sac capsulaire comprennent des corps élastiques ou magnétiques.

18. Dispositif selon la revendication 16,
**caractérisé en ce qu'**une détermination du déplacement du sac capsulaire s'effectue par mesure de distance (67).

19. Dispositif selon la revendication 18,
**caractérisé en ce que** la mesure de distance s'effectue entre un point fixe situé dans l'oeil (65) et un point situé sur le sac capsulaire (64).

20. Dispositif selon l'une des revendications précédentes,
**caractérisé en ce que** le système d'acquisition de données comprend des structures de règles hiérarchiques, des structures centralisées-décentralisées, des systèmes fonctionnant à l'aide d'algorithmes adaptatifs ou des systèmes fonctionnant à l'aide d'algorithmes d'auto-apprentissage ou une combinaison des structures ou des systèmes précités.

21. Dispositif selon l'une des revendications précédentes,
**caractérisé en ce que** l'alimentation en énergie s'effectue par l'intermédiaire d'un système externe (26) qui est en communication avec le système d'alimentation en énergie (23) disposé dans l'implant.

22. Dispositif selon l'une des revendications précédentes,
**caractérisé en ce que** l'alimentation en énergie fait passer le système d'accommodation dans un état de consommation d'énergie minimale, par exemple dans un état de veille, lorsqu'un seuil de luminance n'est pas dépassé sur le chemin optique.

23. Dispositif selon l'une des revendications précédentes,
**caractérisé en ce que** le système d'alimentation en énergie comprend une alimentation en énergie interne.

24. Dispositif selon la revendication 23,
**caractérisé en ce que** l'alimentation en énergie interne s'effectue par utilisation de ressources endogènes, par utilisation d'énergie lumineuse ou par d'autres dispositifs de stockage d'énergie techniques.

25. Dispositif selon la revendication 24,
**caractérisé en ce que** le système d'alimentation en énergie contient des batteries en tant que dispositif de stockage d'énergie technique.

26. Dispositif selon la revendication 24,
**caractérisé en ce que** le système d'alimentation en énergie contient des piles à combustible pour l'utilisation de ressources endogènes.

27. Dispositif selon l'une des revendications précédentes,
**caractérisé en ce que** le système de fixation (22) permet un centrage radial et axial sur le chemin optique et évite des basculements sur le chemin optique.

28. Dispositif selon la revendication 27,
**caractérisé en ce que** le système de fixation comprend des éléments de fixation destinés au centrage axial et radial sur le chemin optique.
